# EUROPEAN PATENT SPECIFICATION

(11) **EP 2 135 083 B1**
(45) Date of publication and mention of the grant of the patent: **23.10.2013**
(21) Application number: 08794288.4
(22) Date of filing: 12.02.2008
(51) Int. Cl.: C12Q 1/04

(54) **METHOD FOR PHAGE DETECTION**
VERFAHREN ZUM NACHWEIS VON PHAGEN
PROCEDE POUR LA DETECTION DE PHAGES

(30) Priority: 12.02.2007 US 900900 P; 08.03.2007 US 905707 P; 05.07.2007 US 958406 P; 03.01.2008 US 18789
(43) Date of publication of application: 23.12.2009
(73) Proprietor: Charm Sciences Inc., Lawrence, MA 01843-1032 (US)
(72) Inventor: SALTER, Robert, S., Reading, MA 01867 (US); DURBIN, Gregory, W., Northboro, MA 01532 (US)
(74) Representative: Webb, Andrew John
(86) International application number: PCT/US2008/001833
(87) International publication number: WO 2008/143722

(56) References cited:
- WO-A-01/79528
- WO-A-94/28179
- WO-A-98/48042
- US-A- 5 527 667
- US-A- 5 605 812
- US-A- 5 965 453
- HUSIMI ET AL: "Cellstat-a continuous culture system of a bacteriophage for the study of the mutation rate and the selection process at the DNA level" REV. SCI. INSTRUM,, vol. 53, no. 4, 1 April 1982 (1982-04-01), pages 517-522, XP008119131
- LINDEMANN BJORN F ET AL: "Evolution of bacteriophage in continuous culture: A model system to test antiviral gene therapies for the emergence of phage escape mutants" JOURNAL OF VIROLOGY, vol. 76, no. 11, June 2002 (2002-06), pages 5784-5792, XP002570561 ISSN: 0022-538X
- SCHWIENHORST A ET AL: "Growth kinetics of a bacteriophage in continuous culture." BIOTECHNOLOGY AND BIOENGINEERING 20 APR 1996, vol. 50, no. 2, 20 April 1996 (1996-04-20), pages 217-221, XP002570562 ISSN: 0006-3592
- HUSIMI ET AL: "Selection and evolution of bacteriophages in cellstat" ADVANCES IN BIOPHYSICS, UNIVERSITY OF TOKYO PRESS, TOKYO, JP, vol. 25, 1 January 1989 (1989-01-01), pages 1-43, XP023445658 ISSN: 0065-227X [retrieved on 1989-01-01]
- HUSIMI ET AL.: 'Cellstat-a continuous culture system of a bacteriophage for the study of the mutation rate and the selection process at the DNA level' REV. SCI. INSTRUM. vol. 53, no. 4, April 1982, pages 517 - 522, XP008119131
- DUPONT QUALICON: 'DuPont-StatMedia soluble packets' 2006, pages 1 - 4, 6, XP008121594
- DUPONT: 'Elvanol-as a Binder/Film Former', [Online] 2004, XP008119133 Retrieved from the Internet: <URL:http://www2.dupont.com/Elvanol/en_US/a ssets/downloads/elvanol_in_water_based_adhe sives.pdf>

## Description

Bacteriophage (phage) are viruses that infect bacteria. Phage can be grouped by the types of bacteria they infect. For example, certain types of phages, referred to as coliphage, infect coliform bacteria. The lytic cycle of coliphage replication cause the cell to lyse and release its contents, including intracellular enzymes.

In environmental water and some food samples the presence of E. coli is an indicator of fecal matter. The Environmental Protection Agency interprets the presence of coliphage in groundwater as an indicator of the presence of fecal matter that is equivalent to detection of E.coli and Enterococci. The equivalence of coliphage as an indicator reflects the idea that the presence of viruses to coliform bacteria must indicate the current or prior presence of coliform bacteria. In foods such as produce, coliphage detection may be useful as an indicator of hygienic production and cleanliness.

Like bacteria, such as pathogenic bacteria, found in fecal matter, viruses, such as enteric viruses, found in fecal matter can pose health risks for humans. Coliphage are an extensive and diverse group of viruses that include medium and large DNA viruses, small genome DNA viruses, and single and double stranded DNA and RNA viruses. Many coliphage are of roughly the same size, molecular weight and nucleic acid contents as enteric viruses and they occur in larger numbers. Coliphage, therefore, can be used as surrogate indicators for the possible presence of human pathogenic viruses.

We describe herein easy-to-use systems, methods and apparatuses for rapidly qualitatively and quantitatively detecting phage, particularly coliphage.
According to the invention there is provided a method for detecting a bacteriophage in a sample as an indicator of a hygienic quality of the sample in accordance with claim 1 in which a combination of sample, growth nutrients for a fecal bacteria, intracellular enzyme induction agent and an indicator for the presence of that intracellular enzyme is inflowed to a second container containing a culture of the fecal bacterial and outflowing from the second container to keep the concentration of bacteria approximately constant. The indicator shows the presence of intracellular enzyme released from the bacteria through lysis by a bacteriophage.

The description also includes systems, methods and apparatus for batch processes not of the present invention. The presence of coliphage in a sample may be detected by mixing the sample with an e-coli culture in a liquid medium providing β-galactosidase and detecting β-galactosidase released from the e-coli after lysis by the coliphage by actions of β-galactosidase on a colorimetric or fluorometric indicator. The e-coli culture is allowed to grow and increase in concentration. Another example of batch processes of a similar nature in a liquid medium, where the indicator is applied to a sample of the supernatant of the medium after filtration is disclosed in WO94/28179. WO94/28179 discloses sensitivity of 2 PFU/ml (2000 PFU/l) after a 6 hour batch process on a single pre-concentrated sample. The method of the present invention can provide sensitivity of 1 or 2 PFU/l i.e a 1000 fold improvement, for a single sample, for a number of discreet samples or for a continuous flow of sample and with detection times as low as 25 minutes.

WO94/28179 also discloses an assay where the culture is on agar. Other examples of batch processes are disclosed in WO81/79528 and WO98/48042.

Figure 1 shows the PocketSwab style format test unit 8 with media tablet. 21 and E.coli tablet 22 in the bottom vial 4.

Figure 2 shows the swab 1 and swab tip 3 removed from the test unit 8.

Figure 3 shows the bottom vial 4 separated from the swab body 7.

Figure 4 shows the bottom vial 4 with media tablet 21 and E.coli tablet 22.

Figure 5 is a cross section of the bottom vial 4. Also shown is an optional niblet 5 containing liquid for rehydrating tablets 21 and 22.

Figure 6 shows the PocketSwab style device 8 with swab tip puncturing through the top seal 12 of the vial 4 and contacting the top seal 13 of the optional niblet 5 and the seal above a dried media/E.coli mixture 15 in the bottom of the vial 4.

Figure 7 is a cross-section with swab tip puncturing through the top seal 12 of the vial 4 and contacting the top seal 13 of the optional niblet 5 and the seal above a dried media/E.coli mixture 15 in the bottom of the vial 4.

Figure 8 shows the swab tip 3 puncturing the top seal 13 of the optional niblet.

Figure 9 is a cross-section showing the swab tip 3 puncturing the top seal 13 of the optional niblet.

Figure 10 is a cross-section showing the liquid from the niblet rehydrating the media/culture mixture 15 in the bottom of the vial 4.

Figure 11 is a flow diagram showing the flow of sample, media and culture, feedback loops and pumps.

We describe herein growing an E. coli culture to an appropriate concentration. The culture is then freeze dried. Freeze drying conditions can be chosen to enhance cell stability. The freeze dried culture, can be supplied to the end-user in combination with appropriate freeze dried media. The media can contain one or more growth indicators, such as a colorimetric and/or fluorogenic enzyme substrates. The culture can be supplied to the end-user either mixed with the media or in separate containers or in separate tablets.

When water samples are to be tested for fecal contamination the sample can be used to rehydrate the E. coli culture and media. The culture and media can be packaged separately or together in dissolvable bags that, upon contact with a liquid such as a liquid sample, or upon contact with a liquid and after heating (incubation), the packaging dissolves allowing the sample to contact the culture and the media. The dissolvable bags can be provided to the user within a variety of possible containers. During incubation, if coliphage is present in the water sample, they will infect the E. coli, form multiple copies, and subsequently lyse the E. coli cells. Lysis of the E. coli cells releases intracellular enzymes such as β-galactosidase. When an appropriate indicator reagent, such as colorimetric and/or fluorogenic enzyme substrates, are included in the media, a detectable change occurs, for example via the enzymatic action of β-galactosidase on the indicator reagent. Such indicators can be used alone or in combination to provide multiple test interpretation options, for example combining colorimetric and fluorogenic indicators. If coliphage are not present then indicator release into solution is relatively slow and no change will be observed during the testing period; the sample will appear turbid as the bacteria multiply and detectable changes (due to indicator reactions) are much slower as compared to if the bacteria were lysed by phage. When bacteria are lysed detection times are in the range of about 2 hours to about 8 hours, for example 4 hours.

The method and apparatus can be provided to customers in an easy-to-use kit form for a variety of applications. Such kits can include bottles for testing, media, culture, saline, and a control. The media and culture can be in dried and in tablet or powdered from. Dissolvable bags can be used with either powders or tablets to prevent human contact.

One application is as an indicator for fecal contamination in groundwater. Other suitable matrices for testing include produce (fruits and vegetable), recreational (swimming) water, farm ruminant, water effluents, waste water and shell fish. Generally, the method and apparatuses can provide users with a rapid, easy-to-use method of measuring the hygienic quality of a sample.

Examples include qualitative and quantitative (most probable number-MPN or plaque forming units -pfu) results. For qualitative testing, a solution in a single container can be used and changes observed in that container. Container sizes can be varied including, for example, standard sample sizes such as 0.5 ml, 1 mL and 100 mL sample sizes. Smaller sample sizes can be accommodated in single service devices such as the POCKETSWAB (POCKETSWAB is a registered trademark of Charm Sciences, Inc. Lawrence, MA) style test device. Particularly for larger sample sizes, such as 100 mL, dissolvable containers, such as dissolvable bags or pouches, may be useful. Such dissolvable containers include those made from polyvinyl alcohol film. Such containers can be water soluble and can be used to package either media or culture separately or as a mixture. The bags can be dissolvable upon contact with water or, alternatively, be dissolvable upon contact with water above a certain temperature. For testing the dissolvable bags can be placed in an appropriate container, or delivered to the end-user in an appropriate container. When the sample is added to the container, or after heating, the bag will dissolve. Dissolution of the bag will allow the media and/or culture to contact the sample. Possible packaging material includes ELVANOL (ELVANOL is a registered trademark of E. I. DU PONT DE NEMOURS AND COMPANY CORPORATION DELAWARE). The dissolvable bag can be used to limit or prevent human contact with test materials including the media and culture. Such contact being through skin or inhalation of powder plume when poured.

For quantitative results, a variety of MPN formats are useful including traditional test tube division and compartmenting devices, such as the QUANTI-TRAY (QUANTI-TRAY is a registered trademark of Idexx Laboratories, Westbrook, Maine) and SIMPLATE (SIMPLATE is a registered trademark of Idexx Laboratories, Westbrook, Maine) devices. Another format includes adding a gelling agent, such as alginate, such as that described in U.S. Patent Serial No. 5,605,812, incorporated herein by reference. The gellable material can be included with the media and culture in a plastic bag along with a solid grid-like device, matrix, such as a plastic grid. Mixing of the sample in the bag with the media and culture will separate the mixture into compartments within the solid grid. Detectable changes will then be observable in separate grids providing the ability to quantitate.

An aspect according to the invention includes a continuous flow method that can be used to monitor a water supply or test multiple samples as defined by the claims. Such methods can provide customers, for example water authorities, a rapid, easy-to-use method of continuously monitoring the hygienic quality of a sample, a series of sample or a continuous flow of material to be tested. The method can include combining, within a first container, a sample with a media to form a first admixture, the first container having an outlet, the media comprising nutrients for the growth of E.coli, induction agents and an indicator for the presence of an E.coli enzyme, such as β-galactosidase. The first admixture flows, for example via a pump, into a second container to form a second admixture. The second container can be continuously mixing or stirring. The second container includes an E.coli culture undergoing exponential growth, a second container inlet and a second container outlet. The second admixture is removed from the second container at approximately the same rate at which the first admixture flows in. In addition, the E.coli culture is being removed at a rate approximately equivalent to the rate at which said E.coli is dividing to maintain a stable concentration range and stable growth phase, such as log phase. The second admixture is incubated within the second container to facilitate the growth of the culture. The optical density of the second admixture is monitored to provide feedback and thereby control, such as through a signal to a pump, the flow rate of the first admixture into the second container and the removal rate of the second admixture from the second container. If coliphage is present in the first container then when the coliphage enters the second container the E.coli therein will be infected and will be lysed. Intracellular enzyme that is released by the lysis can be measured by the affect of such enzyme on the indicator. The presence of coliphage in the sample is used as an indicator of fecal contamination within the sample. Additionally, the growth rate of the phage is faster than the replication rate of the E.coli and, therefore, the E.coli will lyse faster than they replicate. The resulting rapid decrease in absorbance and a shutdown of the flow through system can be used as an indicator just as fluorescence and/or color changes are used as indicators.

Measurements such as optical density, fluorometric, colorimetric measurements can be taken directly from the second admixture or can be taken after the second admixture flows out of the second container. Flow out of the second container can be to waste and can include a diversion to measuring instrumentation such as fluorometers, spectrophotometers and the like. Examples of appropriate flow control mechanisms include various pumps and valves such as a throttling valve. Such flow control mechanisms can be, in a closed system, before or after any of the containers described. Such flow control devices can also be located upstream within the source of sample for the system described herein, such as a throttling valve within an upstream higher pressure water delivery pipe.

By adding a sample to a device that includes a culture already in log phase, rapid detection of coliphage can occur, for example in less than 8 hours. In some aspects detection can be in 25 minutes or less.

Certain aspects include flowing sample continuously, through a first container inlet, into a first container and flowing media continuously, through a second container inlet, into a first container. In such a method a continuous flow of sample could be monitored.

### DETAILED DESCRIPTION

Some embodiments utilize somatic or F+ coliphage-induced lysis and release of β-galactosidase from E. coli strain C (CN-13) or other F+ host E.coli strains. A benefit of using these strains is their antibiotic resistance. Contaminated samples containing coliform may have phage resistant forms that could grow and compete with the host bacteria presented. The antibiotics impart competitive growth advantage to the host against contaminated samples. It should be understood, however, that multiple strains of E. coli or enteric bacteria, for example E. coli strain C (ATCC 13706), and mutants thereof that are infected by bacteriophage, and which produce indicators, can be employed.

E.coli may be cultured to a target concentration, stabilized (to prevent leakage of intracellular content) and then freeze dried for storage until ready for use. Culturing can occur in flasks or fermentors. For E.coli strain C (ATCC 13706) the standard nutrient broth recommended by ATCC can be used with 0.5% NaCl. It may also be possible to increase the level of β-galactosidase in the cells by culturing in an environment that promotes β-galactosidase production, for example by culturing in certain carbohydrate-deprived media with the presence of an inducer compound.

After freeze drying either or both the bacteria and media can be tableted to provide an easy-to-use premeasured amount to the end-user. A combination of tablets and freeze dried powder can also be used. For example, tableted E.coli culture and freeze dried, untableted media. Freeze dried media and E.coli can also be simply combined without tableting. Dried media and dried E.coli can also be provided in separate sealed bags. For example, water soluble bags that dissolve upon contact with sample or upon contact with sample and the addition of heat.

The starting concentration of E.coli in the test may be about 10⁴ to about 10⁸ cfu/ml. The sample size must also be considered so that, for example, if the starting test concentration of E.coli is 10⁶ cfu/ml, and the test will be rehydrated with 100 ml, the concentration of freeze dried E.coli provided can be 10⁸ cfu per volume of dried material (such as tablet) so that the final test concentration is 10⁶ cfu/ml. Similarly, when rehydrated with 1 ml, the freeze dried concentration can be 10⁶ cfu per volume dried material. The concentration of E.coli may be reflected in test result time. For example, more concentrated E.coli may provide faster test results. Higher concentrations, however, may make the test less sensitive to low viral content

A water sample can be added to the combination of media and a dried E. coli cell culture that includes a concentration of cells, in the range of about 10⁴ to about 10⁸ cells. If coliphage are present in the water sample they will infect the E. coli, multiply within the E. coli host, and lyse the E. coli cells causing the release of the intracellular material, including enzymes such as β-galactosidase, into the surrounding medium. β-galactosidase in the surrounding medium will react with appropriate enzyme substrate, such as colorimetric, fluorogenic, or both, whereas intracellular β-galactosidase does not pass through an intact cell wall or cell membrane. In addition, generally in conjunction with the color or fluorogenic color development, high concentrations of coliphage, for example greater than 10⁶ cfu/ml, will result in immediate E.coli lysis and the lack of E.coli growth. The lack of E.coli growth will produce a sample that has a clear appearance as compared to the turbidity of the sample in which E.coli is growing in the absence of coliphage. However, in the presence of a high concentration of coliphage, such as in highly polluted samples, the sample may not detectably change and will just become clear. In such a case, the E.coli are lysed quickly before having time to express sufficient β-galactosidase to generate a visible change. Even in such circumstance, the clarity of the sample, as compared to a turbid negative control, will be easily interpreted as a positive result.

A variety of media, that will support the growth of E.coli, are usefully employed in the media/sample reservoir. Generally, non-selective, non-inhibitory, relatively rich media are useful. For E.coli strain C (CN-13) the standard nutrient broth recommended by ATCC can be used with 0.5% NaCl. A goal in selecting media is to enhance target enzyme production, such as β-galactosidase production, and to limit the available carbohydrate, other than the color or fluorescent indicator(s), upon which β-galactosidase can act. To enhance β-galactosidase the media can include synthetic inducers. Two known synthetic inducers of β-galactosidase are isopropyl-β-D-thiogalactoside (IPTG) and methyl-β-D-thiogalactoside (TMG). Other useful media components include: magnesium sulfate and calcium chloride, which may aid the coliphage binding to and infecting E.coli; a relatively rich broth such as brain heart infusion and yeast extract which may provide nutrients to help maintain the cell wall and, thereby, prevent the premature leakage of β-galactosidase in negative samples and negative controls.

Examples of colorimetric reagents which can be employed as substrates for β-galactosidase include O-nitrophenyl-β-D-thiogalactoside (ONPG), 5-bromo-4-chloro-3-indoyl-β-D-galactoside (X-GAL), and chlorophenol red β, D-galactopyranoside (CPRG). Other possible indicators include glucuronidase, α-galactosidase, chlorophenol red B-D-galactopyranoside, glucosidase, escalinase, pH and optical density. If the β-galactosidase is released into the medium, it will cleave the colorimetric reagent and a detectable colored reaction will occur. If no coliphage is present in the water sample, β-galactosidase will not be released into the medium and any color change will be much substantially slower and the sample turbidity will be constant. That is, during the specified time for coliphage detection, substantial color development will not occur in a sample that does not contain coliphage.

It is recognized that there are other enzymes, and there may be new or well known fluorogenic and chromogenic substrates for β-galactosidase and other enzymes, which could provide identification of E. coli with different color or change in color. Thus, the media identification substrate to be used can vary. An example of a useful fluorogenic substrate is 4-Methylumbelliferyl-β-D-galactopyranoside (MUG-GAL). It may also be possible to use other indicators of E.coli growth, or lack thereof, including colorimetric, fluoregenic, pH indicators and oxidation/reduction indicators. Indicators, such as a color change, fluorescence, pH or optical density, can be used to indicate E.coli lysis or change in the E.coli growth pattern.

It may also be possible to combine indicators, such as combining color indicators with fluorogenic indicators. By combining indicators, for example with an indicator that is visible to the eye under ambient light, and an indicator that requires some instrumentation but may be more sensitive, the user can be provided more flexibility. This is particularly feasible when inducing enzyme production, such as galactosidase with IPTG, and using MUG-GAL as a fluorescent indicator of cell lysis. It may be further beneficial to combine the colorimetric indicator X-GAL with the fluorogenic indicator MUG-GAL.

The dried material (bacteria and media) can be supplied to the end-user in a variety of configurations. For larger sample sizes, such as may be appropriate for testing drinking water, a sealed plastic bag or screw top container can be used. Screw top containers can include a vented cap to provide an opening for gas exchange. For smaller sample sizes, a vial-type container can be used, for example, in a POCKETSWAB type device format described in U.S. Patent No. 5,965,453.

One useful container is a transparent, flexible, throwaway, plastic, sealable bag which contains the dried test composition such as the ECOLITE (ECOLITE is a registered trademark of Charm Sciences, Inc. Lawrence, MA) described in U.S. patent No. 5,728,542. To use, the bag is unsealed, and a defined amount, such as for example, 100 ml of the water sample is poured into the bag. The bag is then closed tightly by folding tightly, for example with a wire strip or other sealing means, to form a water tight seal. With the bag closed, the user thoroughly mixes the reagent medium. The bag with the dry medium, dried microbial culture and the water test sample is sealed and incubated at about 37° C ±2° C., for about 2-to about 8 hours depending on instructions provided with the test. Color or other indicator change during that time period indicates the presence of coliphage and, therefore, the presence of fecal contamination. If no change occurs during the testing time, and the sample appears turbid, coliphage is not present in the sample. In a highly contaminated sample in which high concentrations of coliphage quickly lyse the E.coli cells in the culture, the sample may appear clear with no changes, for example no color development.

Rather than have the media and/or culture supplied directly in the container within which testing will be conducted, the media and/or culture can be provided in a separate sealed container such as a tearable container. The media and/or culture can also be supplied in dissolvable bags within, for example, the tearable container. In such an embodiment, the tearable or pealable container or blister pack would be torn and the media added or the dissolvable bag(s) added to a separate sealable plastic bag, or screw top solid container, such as a screw top container that can hold approximately 100 mL to 200 mL of liquid, within which has been added the liquid sample to be tested. The dissolvable bag is particularly useful for use with the culture to prevent or limit human contact with the culture. In addition, tablets can be used, with or without dissolvable containers. The tablets can contain freeze dried E.coli culture and/or media. The tablets can be added first to saline or, if culture, to media and rehydrated and/or incubated before adding as a liquid.

Larger sample sizes for which quantitation is desired, such as 100 mL samples, after rehydration, can be aliquoted into multiple containers for MPN analysis. For example the sample can be poured into a device such as that known as the QUANTI-TRAY and described in Naqui et al., U.S. Pat. No. 5,518,892. The apparatus features a bag which is designed for receiving a liquid sample and subsequently distributes the liquid sample into separate compartments within the bag so that different aliquots of one or more sizes may be tested. The.quantifying step involves detecting the quantity and quality of the color, or other, change in each compartment, and comparing that quantity and quality to an MPN table.

Using the QUANTI-TRAY format with the herein described phage detection method and device. The presence of turbidity and absence of color, fluorescence, or other indicator, in the developing well of the Naqui device indicates that coliphage is not present in the sample: that coliphage are not present thereby allowing the E.coli culture to grow unimpaired by coliphage. Each compartment of the Naqui invention that clarifies or otherwise changes can be observed and the result in each counted to arrive at a coliphage MPN result. Other smaller volume MPN type devices that may be useful include the SIMPLATE devices and TEMPO (TEMPO is a registered trademark of bioMerieux CORPORATION Chemin de l'Orme Marcy - L'Etoile FRANCE). Users may also simply aliquot by pipetting into multiple test containers prior to test incubation.

Using the POCKETSWAB format the dried material can be provided in two separate tablets: a media tablet and a bacteria tablet. A variety of possibilities for obtaining a sample include removing the vial portion of the POCKETSWAB device and using it to "scoop" a liquid sample into the vial. The vial portion can then be replaced and the swab, or probe, provided with the device used to puncture the membrane seals to contact the sample with the dried or tableted media and culture. Similarly, a sample can be pipetted into the vial. Alternatively, the swab portion of the device containing a liquid, such as a buffer, portion above the tablets can be used to absorb a sample, for example, from a vegetable rinse, spinach leaf or mollusk stomach. In any case, the swab can be used to puncture the various compartments within the vial thereby allowing the buffer-sample mixture to contact the media and culture. When the swab is used to absorb a sample, additional liquid may be required, either from another sealed compartment within the device, for rehydrating the media and culture.

The format of the POCKETSWAB provides the advantage of controlled movement of the probe in a test device that provides physical support for the probe and compartments for storage of reagents and sample addition.

Depending on the disposal and other potential contamination issues involving culturing E.coli, it may be useful to employ a device, such as described in International Patent Publication WO/2006/069053, published June 26, 2006. It may also be useful to include a bactericide and/or viricide to provide the end-user with a convenient method for killing the culture and/or phage after test operation.

In another example of use for pfu/ml quantitation, a gelling agent may be useful, such as available in the COLIGEL (COLIGEL is a registered trademark of Charm Sciences, Inc., Lawrence, MA) format described in U.S. Patent No. 5,605,812. In such an embodiment, coliphage presence will be indicated by detectable change in the gel media. Quantitation may also be possible in such a format by counting the spots, such as spots of color, in the gel.

The methods and devices described herein are largely directed to detecting somatic coliphage in an E.coli culture through the action of the E.coli enzyme β-galactosidase. Those skilled in the art will appreciate that detecting other phages, such as male F+ coliphage, can similarly be used to indicate the presence of the phage susceptible bacteria or the sample environment to which the presence of such bacteria can be related. Similarly, other enzymes and indicators may be usefully detected as indicators of cell lysis.

By utilizing the various methods and devices described herein, detection of a contaminated sample can occur rapidly, for example in less than 8 hours, preferably in less than hour. For example in some conditions a single phage can infect a cell and replicate 100 or more progeny in as little as 10 minutes. After 20 minutes 10,000 progeny could be produced and in 30 minutes 1,000,000 progeny could be produced. Thus, detection can occur in less than about 30 minutes from initial infection. Such rapid detection is achieved by flowing sample continuously into a culture that has been established in log phase growth and detecting the lysis of the cells in the culture by infecting phage. The culture can be grown separately and then added to a container that is maintained at an appropriate temperature for E.coli growth, for example about 37 degrees C (the culture container). In an embodiment the target concentration of E.coli in the test is about 3 x 10⁷ cfu/mL to 8 x 10⁷ cfu/mL. The culture container can be flowably connected to a media/sample reservoir. A pump can be used to pump the contents of the media/sample reservoir into the culture container.

The media/sample reservoir can be fed by a media container that can include a selected, appropriate media. The media/sample reservoir can also be fed by a sample container that can include a sample to be tested. The sample container can also include, instead of the sample, a negative or positive control. An example of a negative control is sterile water. The media/sample reservoir can be flowably connected to the culture container through an outlet from the media/sample reservoir. In an embodiment of a continuous flow system, in which a stream of sample is provided, the media/sample reservoir can also be connected to a sample source for example through inlets into the media/sample reservoir. Such an inlet can be used to provide sample continuously or, for example, until a positive result is found at which time flow of sample can be stopped either automatically or manually.

After the culture within the culture container is stabilized at approximately the target cell concentration, sample mixed with media can be added. During incubation, if coliphage is present in the water sample, they will infect the E. coli, form multiple copies, and subsequently lyse the E. coli cells. Lysis of the E. coli cells releases intracellular enzymes, such as induced enzymes, including β-galactosidase. When an appropriate indicator reagent, such as colorimetric and/or fluorogenic indicators, are included in the media, a detectable change occurs, for example via the enzymatic action of β-galactosidase on the indicator reagent.

It may be further beneficial to combine a colorimetric indicator such as X-GAL with a fluorogenic indicator such as MUG-GAL. For example, it may be possible to enhance fluorescent results, particularly the fluorescent color in negative results, through the use of a colorimetric indicator, such as X-GAL, with a fluorescent indicator, such as MUG-GAL. If coliphage are not present, then the indicator release into solution is relatively slow and no change will be detected. If coliphage are present, then bacteria are lysed and the enzyme is released into the outer media where it can act on the indicator.

As media/sample flows from the media/sample reservoir into the culture container, outflow from the culture container removes sufficient amount of culture to maintain the culture within the culture container at a relatively steady stage of growth and concentration. That is, as the E.coli are dividing, the rate of flow of media and sample into the culture container, and the rate of flow of culture out of the culture container, are adjusted to maintain the concentration at a relatively steady concentration and in log phase of growth. The culture container can be sealed to prevent contamination, in which case the in flow rate is approximately equal or exactly equal to the outflow rate. Monitoring of the growth of the cells can be by methods known to those skilled in the art, such as optical density.

The outflow can be to a waste container. The outflow can also flow into an intermediate container between the culture container and the waste container. The intermediate container can be positioned relative to a spectrophotometer so that the spectrophotometer can be used to determine the optical density (OD) of the material which is used as an indicator of the culture concentration. The appropriate OD can be maintained, for example OD520, by a feedback loop that controls a pump or other flow control device such as a control valve, for example a peristaltic pump, positioned between the media/sample reservoir and the culture container or anywhere in the closed system. Similarly, the signal from the material, such as fluorescence and/or colorimetric, can be determined at the intermediate container by an instrument such as a fluorometer, colorimeter or spectrophotometer. By having the media/sample reservoir, culture container and waste container flowably connected, a continuous flow of samples for detection can be achieved. For example, a water authority could continuously sample its water supply by diverting water samples into the system for continuous detection.

After a positive sample is detected a continuous flow system must be cleaned. Some embodiments include a clean in place system to allow the entire flow path to be cleaned before new sample is introduced. Other embodiments include a disposable flow path that can be replaced periodically and/or when a positive sample flows through and thereby contaminates the system.

The methods and devices described herein are largely directed to detecting somatic coliphage in an E.coli culture through the action of the E.coli enzyme β-galactosidase. Those skilled in the art will appreciate that detecting other phages, such as male F+ coliphage, by changing host bacteria and selective media components, can similarly be used to indicate the presence of the phage susceptible bacteria or the sample environment to which the presence of such bacteria can be related. Similarly, other enzymes and indicators may be usefully detected as indicators of cell lysis. In addition, there will be a measurable OD decrease as the cells lyse and in an automated system this will reduce the inflow rate.

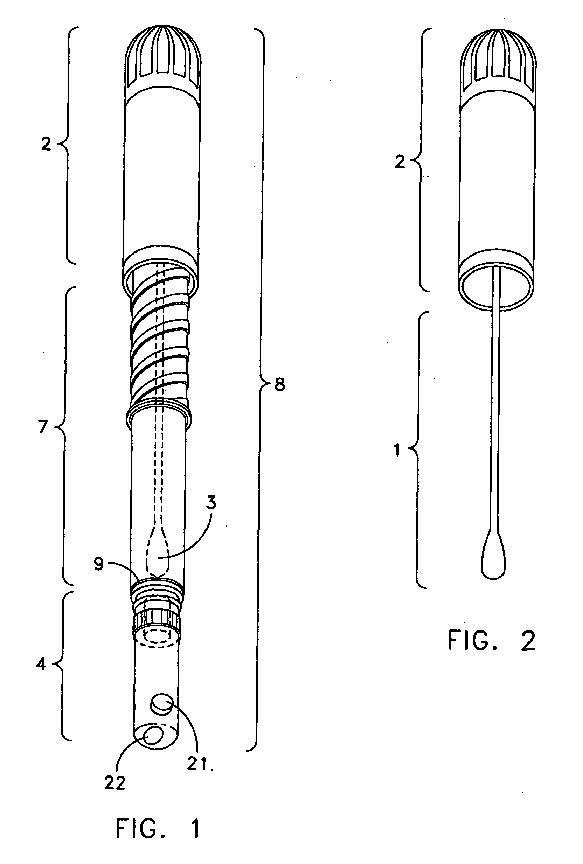

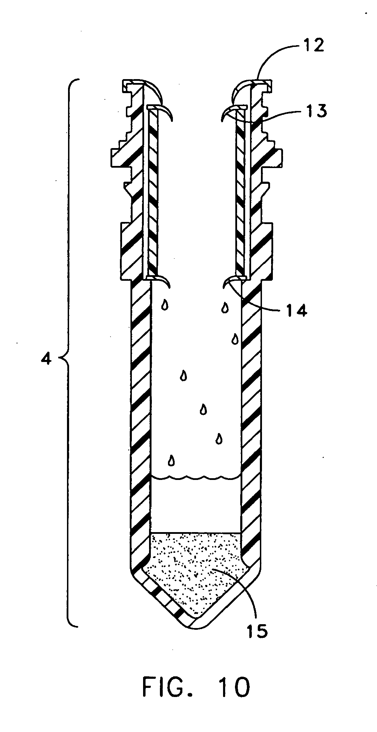

Figures 1-10 show a PocketSwab style test device. Such a device can be used for small sample sizes of about 0.5 mL to about 1 mL. For larger sample sizes, for example 100 mL, a screw top container or plastic bag device can be used.

Figure 1 shows the PocketSwab style format test unit 8 with media tablet 21 and E.coli tablet 22 in the bottom vial 4. Figure 2 shows the swab 1 and swab tip 3 removed from the test unit 8. The swab tip 3 can be used to absorb a sample, for example from a spinach leaf or other material to be tested. In some embodiments the swab tip 3 is unnecessary, such as when the vial 4 is used to "scoop" a sample or if the sample is pipetted into the vial 4, in such embodiments the swab 1 can be simply a probe to puncture the seals in the vial and need not include an absorbent tip.

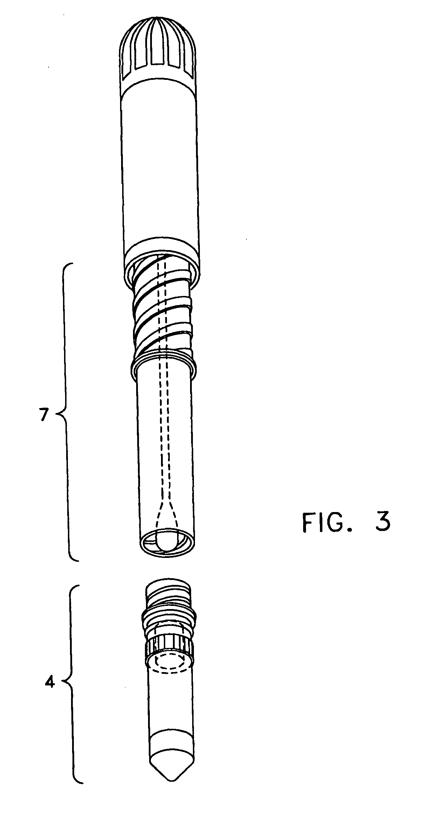

Figure 3 shows the bottom vial 4 portion separated from the swab body 7. The vial portion can be threadably attached to the swab body 7 for easy removal and use either to "scoop" a sample or to allow access to the media/culture in the vial 4 other than by using the probe 1. In embodiments in which a sample is pipetted into the vial 4, no probe is required and the seal can be punctured with, for example, a pipette tip.

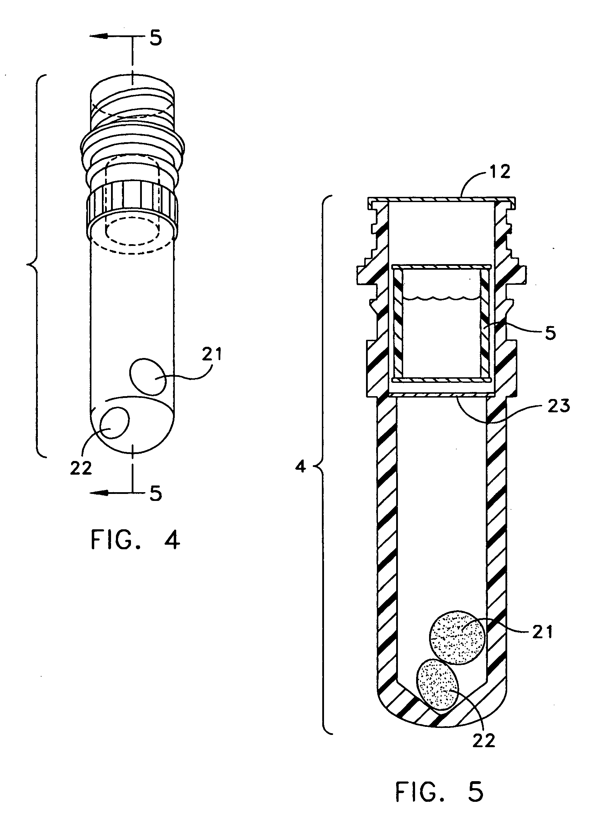

Figures 4 and 5 show the bottom vial 4 portion with media tablet 21 and E.coli tablet 22. The optional niblet 5 is also shown. If additional liquid, such as buffer, is not required to rehydrate the media/culture tablets 21, 22 or powdered media/culture mixture 15, the niblet 5 can be excluded. If the niblet 5 is excluded then a foil seal can be situated above the tablets 21, 22 or media 15. As shown in figure 5, this seal 23 can be the only seal so that a sample can be "scooped", pipetted or poured into the area between seal 12 and seal 23. To do so, seal 12 must be punctured before sampling or, alternatively, seal 12 can be excluded. If seal 12 and niblet 5 are excluded then after sampling seal 23 can be punctured to allow the sample to mix with the reagents.

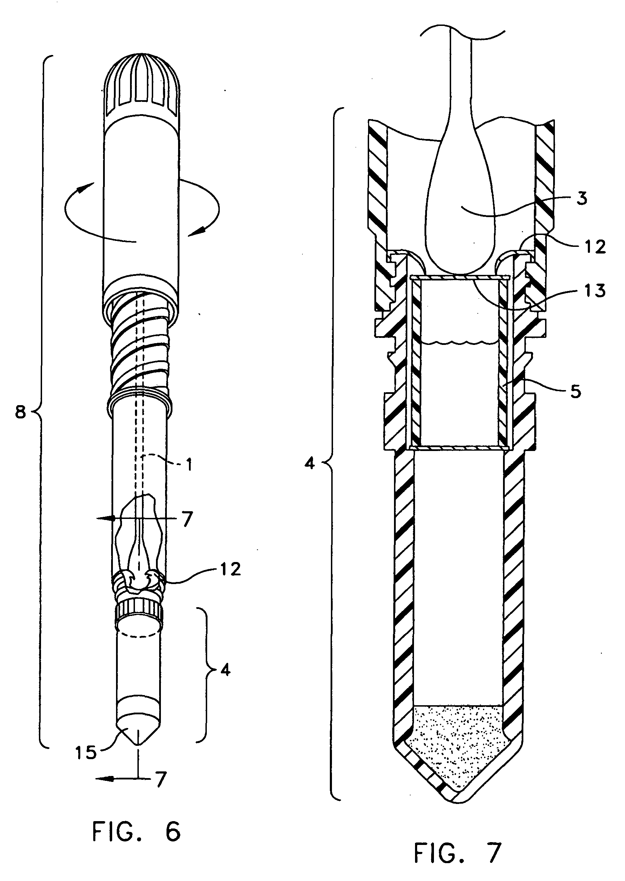

Figure 6 shows the PocketSwab style device 8 with swab tip puncturing through the top seal 12 of the vial 4 and contacting the top seal 13 of the optional niblet 5 and the seal above a dried media/E.coli mixture 15 in the bottom of the vial 4.

Figure 7 is a cross-section with swab tip puncturing through the top seal 12 of the vial 4 and contacting the top seal 13 of the optional niblet 5 and the seal above a dried media/E.coli mixture 15 in the bottom of the vial 4.

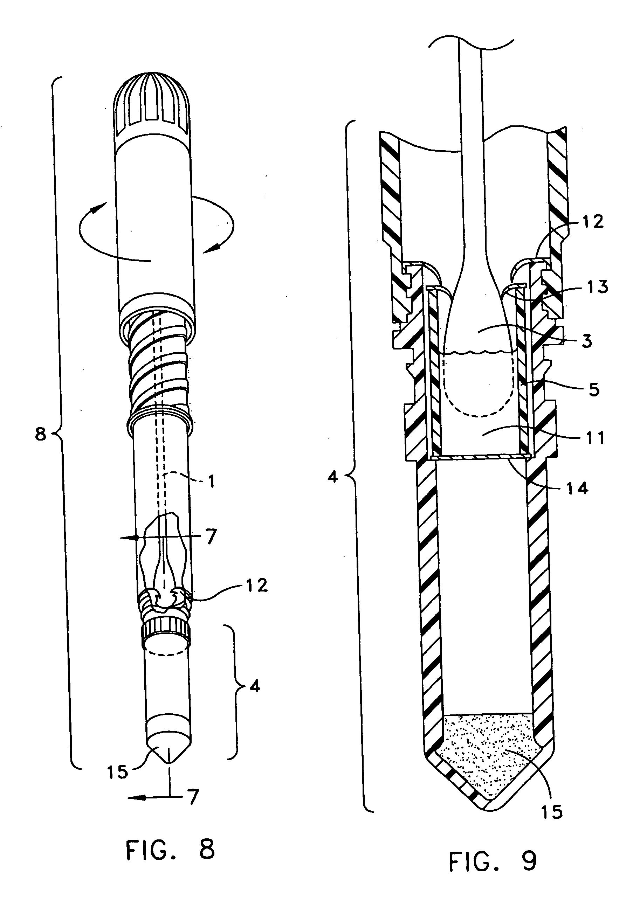

Figures 8, 9 and 10 show the swab tip 3 puncturing through the various seals including the optional niblet seals to allow the liquid from the niblet to rehydrate the media/culture mixture 15 in the bottom of the vial 4.

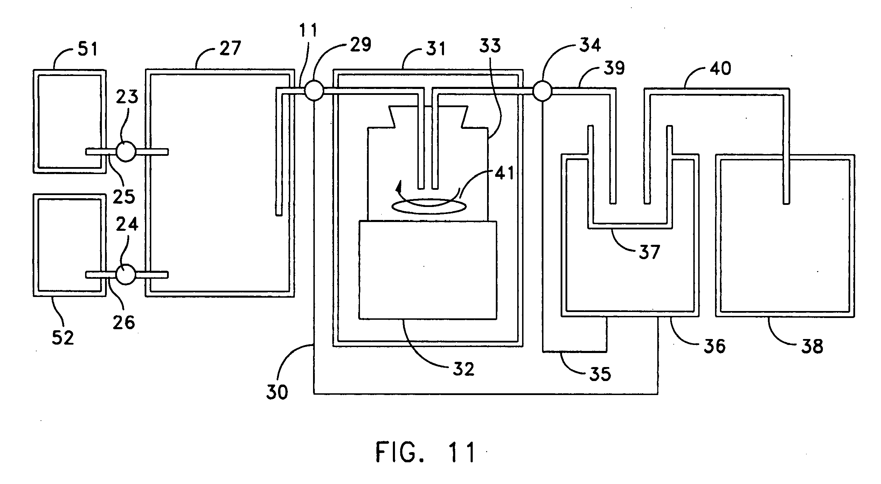

Figure 11 is a flow diagram showing the flow of sample, media and culture through an example of a continuous flow system. Media can flow from media container **51**, through line **25** into reservoir **27**. Sample can flow from sample container **52**, through line **26** into reservoir **27**. Pump **23** and pump **24** can be used to control the flow from sample container **52** and media container **51** into reservoir **27**. Sample container **52** can be replaced by a valve, or other means, that diverts the flow of sample from its normal path into the system for testing. Within reservoir **27** media and sample can be mixed. The media/sample mixture can flow through line **28** into culture container **33**. Flow control device **29** can control the flow of media/sample from reservoir **27** into culture container **33**. Culture can flow, controlled by flow control device **34**, out of culture container **33**, through line **39** into testing container **37**. Flow can also proceed from testing container **37** through line **40** to waste container **38**. Flow control devices **34** and **29** can be controlled by feedback from measuring device **36**. In a closed system pumps **34** and **29** are redundant and not both be needed and, in a closed system, could be placed in a variety of locations. Particularly in a closed system, the flow from reservoir **27** to culture container **33** and out from culture container **33** to waste container **38** can be maintained at the same rate. Culture can be maintained within culture container **33** by incubator **31**. Mixing of culture, media and sample within culture container **33** can be facilitated by magnetic stirrer **32** and stir bar **41**. Mixing can also occur within reservoir **27**, media container **51** and sample container **52**.

### EXAMPLES

### Example 1

### Media Formulation

The following formulation was found useful for an individual 100 ml water quality test (2.8 grams(g) media per test):
2 g LB broth; 0.01g IPTG; 0.3g Brain Heart Infusion; 0.5 tryptone; 0.007g CaCl2; 0.003g MgSO4; 0.025g ONPG ***or*** 0.008g Xgal
For the PocketSwab format a tablet containing 23milligrams (mg) of the above media was used.

### E.coli Culture formulation

Culturing was in a flask. E.coli strain C (ATCC 13706) was used and grown in the standard nutrient broth recommended by ATCC with 0.5% NaCl.

After culturing the E.coli culture was mixed 1:1 with 24% sucrose solution before freeze drying. After freeze drying the following material was combined with the freeze dried culture and the mixture tableted.

| Material | Percentage | Grams |
|---|---|---|
| Dry Emdex (60-100) | 82.75 | 16.55 |
| Maltrin M-510 | 10.00 | 2.0 |
| PEG 8000 | 4.00 | 0.80 |
| Poly XL | 3.00 | 0.60 |
| Mg stearate | 0.25 | 0.05 |

Above formulation mixed for 5 minutes with bulk freeze dried E.coli culture and sifted using a sieve size of 60-E.

### Example 2

The following results are from an experiment using various dilutions of somatic coliphage. Media used, per 100 mL, was 2 g LB broth; 0.01g IPTG; 0.3g Brain Heart Infusion; 0.007g CaCl2; 0.003g MgSO4; 0.025g ONPG. In addition, a variety of concentrations of tryptone (tryp) (0.3 grams, 0.5 grams, and 0.75 grams) were used to assess the affect on test performance.

E.coli culture was E.coli strain C (ATCC 13706) grown in the standard nutrient broth recommended by ATCC with 0.5% NaCl.
PocketSwab format was used with
neg: no color development, turbid= negative for coliphage
clear: no color development, no turbidity-positive for coliphage
(+/-): weak color development=positive for coliphage
(+): positive color development-positive for coliphage

0.5 mL samples were pitpetted into the bottom vial of the PocketSwab style device.

Results were as follows:

| SAMPLE | Phage/test | 0 tryp | 0.3 g tryp | 0.5 g tryp | 0.75 g tryp |
|---|---|---|---|---|---|
| neg control | 0 | neg | neg | neg | neg |
| 10⁻⁷ | 1 | neg | (+/-) | + | neg |
| 10⁻⁶ | 10 | + | + | + | + |
| 10⁻⁵ | 100 | + | + | + | + |
| 10⁻⁴ | 1,000 | + | + | + | + |
| 10⁻³ | 10,000 | + | + | + | + |
| 10⁻² | 100,000 | + | + | + | + |
| 10⁻¹ | 1,000,000 | (+/-) | (+/-) | (+/-) | (+/-) |
| Undiluted | 10,000,000 | clear | clear | clear | clear |

### Example 3

The following results are from an experiment using various dilutions of somatic coliphage in a 100 mL sample. Tests were incubated for 3 hours at 37 degrees C in a 160 mL screw top solid container with a vent to allow oxygen transfer. Media used per 100 mL was 2 g LB broth; 0.01g IPTG; 0.3g Brain Heart Infusion; 0.007g CaCl2; 0.003g MgSO4; 0.008g Xgal. In addition, a 0.5 grams tryptone was added to assess the affect on test performance. Tests were incubated for 3 hours at 37 degrees C in a screw top solid container with a vent to allow gas exchange.
E.coli culture was E.coli strain C (ATCC 13706) grown in the standard nutrient broth recommended by ATCC with 0.5% NaCl.
neg: no color development, turbid= negative for coliphage
clear: no color development, no turbidity=positive for coliphage
(+/-): weak color development=positive for coliphage
(+): positive color development=positive for coliphage

Results were as follows:

| SAMPLE | Phage/test | 0 tryp | 0.5 g tryp |
|---|---|---|---|
| neg control | 0 | neg | Neg |
| 10⁻⁸ | 0.1 | neg | Neg |
| 10⁻⁷ | 1 | (+/-) | + |
| 10⁻⁶ | 10 | + | + |
| 10⁻⁵ | 100 | + | + |
| 10⁻⁴ | 1,000 | + | + |
| 10⁻³ | 10,000 | + | + |
| 10⁻² | 100,000 | + | + |
| 10⁻¹ | 1,000,000 | (+/-) | (+/-) |
| Undiluted | 110,000,000 | clear | Clear |

### Example 4 - Continuous Flow System

### Media Formulation

2 g LB broth; 0.01g IPTG; 0.3g Brain Heart Infusion; 0.5 tryptone; 0.007g CaCl2; 0.003g MgSO4; 0.025g ONPG ***or*** 0.008g Xgal; 0.01 g naladixic acid

### E.coli Culture formulation

Culturing was in a flask. E.coli strain C (CN-13) was used and grown in the standard nutrient broth recommended by ATCC with 0.5% NaCl.

After culturing the E.coli culture was mixed 1:1 with 24% sucrose solution before freeze drying. After freeze drying the following material was combined with the freeze dried culture and the mixture tableted.

| Material | Percentage | Grams |
|---|---|---|
| Dry Emdex (60-100) | 82.75 | 16.55 |
| Maltrin M-510 | 10.00 | 2.0 |
| PEG 8000 | 4.00 | 0.80 |
| Poly XL | 3.00 | 0.60 |
| Mg stearate | 0.25 | 0.05 |

Above formulation mixed for 5 minutes with bulk freeze dried E.coli culture and sifted using a sieve size of 60-E.

### Example 5- Continuous Flow System

An alternative media formulation to that of Example 4 is as follows: 1.4g media per test:
0.7g LB broth; 0.01g IPTG; 0.5 tryptone; 0.007g CaCl2; 0.06g MgSO4; 8 mg Xgal; 7 mg MUG-GAL; 0.01g naladaxic acid; and 0.06g sucrose.

### Example 6 - Continuous Flow System

The following tables include results from two different experiments using the culture from Example 4 and the media from Example 5.

Table 1 includes results from experiments with negative control sterile water. The initial time entry of -180 minutes represents the time for the culture to grow in log phase to the appropriate concentration for log phase growth: 0.5 x 10⁶ to 0.5 x 10⁸ per mL. As is shown in the table, the negative control flowed into the culture for 125 minutes. During that time the sample did not visually fluoresce. Although visual observation of fluorescence was used, it is expected that in an automated, continuous flow system, fluorescence will be measured by an instrument. After a steady flow rate of 180 mL/hr was reached, the OD 520 nm was relatively stable and, therefore, no adjustment to the flow rate was made for the duration of the experiment.

**Table 1**

| Time (minutes) | Flow Rate mL/hr | Total Volume of Sample Tested | OD 520 nm | Visual Fluorescence |
|---|---|---|---|---|
| -180 | 0 | 0 | 0 | negative |
| 5 | 234 | 19.5 | 0.259 | negative |
| 20 | 246 | 80 | 0.237 | negative |
| 35 | 216 | 135 | 0.221 | negative |
| 50 | 180 | 184 | 0.199 | negative |
| 65 | 180 | 225 | 0.222 | negative |
| 80 | 180 | 270 | 0.222 | negative |
| 95 | 180 | 315 | 0.219 | negative |
| 110 | 180 | 360 | 0.227 | negative |
| 125 | 180 | 405 | 0.236 | negative |

Table 2 includes results from experiments with a positive sample (∼1-2 pfu/liter). The initial time entry of -180 minutes represents the time for the culture to grow to the appropriate concentration for log phase growth. As is shown in the table, visible fluorescence began to be observed at 315 minutes. From that point on fluorescence got stronger. Although visual observation of fluorescence was used, it is expected that in an automated, continuous flow system, fluorescence will be measured by an instrument.

After a steady flow rate of 180 mL/hr was reached, the OD 520 nm increased steadily until the flow rate was increased to 240 mL/hr to offset the culture growth. After the flow rate was increased, the OD was reduced. In this experiment the flow rate was adjusted manually. It is expected that in an automated, continuous flow system, flow rate rate will be automatically adjusted based on target OD 520 readings. In addition, as can be seen by the last 2 readings at times 330 and 335, the OD dropped and flow rate stopped as the fluorescence strengthened. Thus, in addition to fluorescence, the flow rate and OD reductions could also be used as indicators of a positive result for coliphage.

**Table 2**

| Time | Flow Rate mL/hr | Total Volume of Sample Tested | OD 520 nm | Visual Fluorescence |
|---|---|---|---|---|
| -180 | 0 | 0 | 0 | negative |
| 5 | 234 | 19.5 | 0.298 | negative |
| 20 | 192 | 76 | 0.278 | negative |
| 35 | 192 | 123 | 0.263 | negative |
| 50 | 180 | 169 | 0.253 | negative |
| 65 | 180 | 210 | 0.260 | negative |
| 80 | 180 | 255 | 0.264 | negative |
| 95 | 180 | 300 | 0.247 | negative |
| 110 | 168 | 342 | 0.252 | negative |
| 125 | 168 | 384 | 0.250 | negative |
| 155 | 168 | 468 | 0.269 | negative |
| 185 | 168 | 552 | 0.272 | negative |
| 225 | 168 | 636 | 0.307 | negative |
| 255 | 168 | 720 | 0.354 | negative |
| 285 | 240 | 864 | 0.260 | negative |
| 315 | 204 | 966 | 0.207 | slight fluorescence |
| 320 | 204 | 977 | 0.193 | +fluorescence |
| 325 | 204 | 988 | 0.173 | +fluorescence |
| 330 | 66 | 993 | 0.119 | ++fluorescence |
| 335 | 0 | 993 | 0.110 | strong fluorescence |

## Claims

1. A method for detecting a bacteriophage in a sample as an indicator of a hygienic quality of the sample, the method comprising the steps of:
a. combining the sample and a media in a first container to form a first admixture, the first container having a first container outlet, the media comprising: (i) nutrients for the growth of a fecal bacteria; (ii) an intracellular enzyme induction agent capable of inducing the production, within the fecal bacteria, of an intracellular enzyme; and (iii) an indicator for the presence of the intracellular enzyme, the intracellular enzyme being released from the bacteria through the lysis of the bacteria by a bacteriophage;
b. inflowing the first admixture to a second container to form a second admixture, the second container comprising a culture of the fecal bacteria, a second container inlet and a second container outlet;
c. outflowing the second admixture from the second container, wherein the inflowing to the second container and the outflowing from the second container are occurring at approximately the same rate, and wherein the fecal bacteria is being removed at a rate approximately equivalent to a rate at which the bacteria is dividing;
d. incubating the second admixture within the second container;
e. monitoring the number of the bacteria in the second admixture;
f. controlling a flow rate of the first admixture into the second container, and the removal rate of the second admixture from the second container, by reference to the number of bacteria in the second admixture; and
g. measuring the presence of the intracellular enzyme by the affect of the enzyme on the indicator,
wherein the presence of the intracellular bacterial enzyme in the second admixture indicates the presence of the bacteriophage in the sample and wherein the presence of the bacteriophage is an indicator for the poor hygienic quality of the sample.

2. A method according to claim 1 wherein the fecal bacteria comprises *Escherichia coli* and the bacteriophage comprises coliphage.

3. A method according to claim 2 wherein the intracellular enzyme induction agent comprises isopropyl-β-D-thiogalactoside.

4. A method according to claim 2 wherein the fecal bacteria comprises *E.coli* in log phase of growth.

5. A method according to claim 2 wherein the indicator comprises a fluorogenic indicator for the presence of β-galactosidase.

6. A method according to claim 2 wherein the indicator is selected from the group consisting essentially of: 4-Methylumbelliferyl-β-D-galactopyranoside; O-nitrophenyl-β-D-thiogalactoside; 5-bromo-4-chloror-3-indoyl-β-D-galactoside; and chlorophenol red B-D-galactopyranoside.

7. A method according to claim 1 wherein the indicator comprises a combination of a colour indicator and a fluorescent indicator.

8. A method according to claim 2 further comprising maintaining the fecal bacteria in a log phase of growth.

9. A method according to claim 1 wherein controlling the flow rate comprises measuring cell growth in the second admixture and providing feedback to a flow control device.

10. A method according to claim 9 wherein controlling the flow rate comprises measuring the optical density of the second admixture and providing feedback to a flow control device.

11. A method according to claim 1 further comprising measuring a detectable change in the outflow from the second container.

12. A method according to claim 1 further comprising measuring a detectable change in the second container.

13. A method according to claim 1 further comprising flowing the sample continuously through a first container inlet and into the first container.

14. A method according to claim 13 further comprising flowing the sample continuously, through a first container inlet into the first container and flowing media continuously through a second container inlet into the first container.

15. A method according to claim 1 wherein the detecting of the bacteriophage as an indicator of the hygienic quality of the sample occurs in less than 8 hours of contacting the sample with the media.

## Patentansprüche

1. Verfahren zum Nachweisen einer Bakteriophage in einer Probe als Indikator einer hygienischen Qualität der Probe, wobei das Verfahren die Schritte umfasst:
a. Vereinigen der Probe und eines Mediums in einem ersten Behälter zum Bilden eines ersten Gemischs, wobei der erste Behälter einen Auslass des ersten Behälters aufweist, wobei das Medium umfasst: (i) Nährstoffe für das Wachstum von Fäkalbakterien, (ii) ein intrazelluläres Enzyminduktionsmittel, das die Erzeugung eines intrazellulären Enzyms innerhalb der Fäkalbakterien induzieren kann, und (iii) einen Indikator bezüglich der Gegenwart des intrazellulären Enzyms, wobei das intrazelluläre Enzym aus den Bakterien durch die Lyse der Bakterien durch eine Bakteriophage freigesetzt wird,
b. Einbringen des ersten Gemischs in einen zweiten Behälter zum Bilden eines zweiten Gemischs, wobei der zweite Behälter eine Kultur der Fäkalbakterien, einen Einlass des zweiten Behälters und einen Auslass des zweiten Behälters umfasst,
c. Ausbringen des zweiten Gemischs aus dem zweiten Behälter, wobei das Einbringen in den zweiten Behälter und das Ausbringen aus dem zweiten Behälter etwa mit der gleichen Geschwindigkeit stattfinden, und wobei die Fäkalbakterien mit einer Geschwindigkeit entfernt werden, die etwa äquivalent zu einer Geschwindigkeit ist, mit der sich die Bakterien teilen,
d. Inkubieren des zweiten Gemischs innerhalb des zweiten Behälters,
e. Überwachen der Anzahl der Bakterien in dem zweiten Gemisch,
f. Steuern der Fließgeschwindigkeit des ersten Gemischs in den zweiten Behälter und der Entfernungsgeschwindigkeit des zweiten Gemischs aus dem zweiten Behälter unter Bezugnahme auf die Anzahl von Bakterien in dem zweiten Gemisch und
g. Messen der Gegenwart des intrazellulären Enzyms durch die Einwirkung des Enzyms auf den Indikator,
wobei die Gegenwart des intrazellulären bakteriellen Enzyms in dem zweiten Gemisch die Gegenwart der Bakteriophage in der Probe anzeigt und wobei die Gegenwart der Bakteriophage ein Indikator für die schlechte hygienische Qualität der Probe ist.

2. Verfahren nach Anspruch 1, bei dem die Fäkalbakterien *Escherichia coli* umfassen und die Bakteriophage Coliphage umfasst.

3. Verfahren nach Anspruch 2, bei dem das intrazelluläre Enzyminduktionsmittel Isopropyl-β-D-thiogalactosid umfasst.

4. Verfahren nach Anspruch 2, bei dem die Fäkalbakterien *E. coli* in der log-Phase des Wachstums umfassen.

5. Verfahren nach Anspruch 2, bei dem der Indikator einen fluorogenen Indikator bezüglich der Gegenwart von β-Galactosidase umfasst.

6. Verfahren nach Anspruch 2, bei dem der Indikator aus der Gruppe, bestehend im Wesentlichen aus 4-Methylumbelliferyl-β-D-galactopyranosid, O-Nitrophenyl-β-D-thiogalacto-sid, 5-Brom-4-chlor-3-indoyl-β-D-galactosid und Chlorphenolrot B-D-galactopyranosid, ausgewählt ist.

7. Verfahren nach Anspruch 1, bei dem der Indikator eine Kombination aus einem Farbindikator und einem Fluoreszenzindikator umfasst.

8. Verfahren nach Anspruch 2, das ferner das Halten der Fäkalbakterien in einer log-Phase des Wachstums umfasst.

9. Verfahren nach Anspruch 1, bei dem das Steuern der Fließgeschwindigkeit das Messen des Zellwachstums in dem zweiten Gemisch und das Bereitstellen einer Rückkopplung zu einer Fließsteuereinrichtung umfasst.

10. Verfahren nach Anspruch 9, bei dem das Steuern der Fließgeschwindigkeit das Messen der optischen Dichte des zweiten Gemischs und das Bereitstellen einer Rückkopplung zu einer Fließsteuereinrichtung umfasst.

11. Verfahren nach Anspruch 1, das ferner das Messen einer nachweisbaren Änderung bei dem Ausfluss von dem zweiten Behälter umfasst.

12. Verfahren nach Anspruch 1, das ferner das Messen einer nachweisbaren Änderung in dem zweiten Behälter umfasst.

13. Verfahren nach Anspruch 1, das ferner das kontinuierliche Strömenlassen der Probe durch einen Einlass des ersten Behälters und in den ersten Behälter umfasst.

14. Verfahren nach Anspruch 13, das ferner das kontinuierliche Strömenlassen der Probe durch einen Einlass des ersten Behälters in den ersten Behälter und das kontinuierliche Strömenlassen des Mediums durch einen Einlass des zweiten Behälters in den ersten Behälter umfasst.

15. Verfahren nach Anspruch 1, bei dem das Nachweisen der Bakteriophage als Indikator der hygienischen Qualität der Probe in weniger als 8 Stunden ab dem Inkontaktbringen der Probe mit dem Medium stattfindet.

## Revendications

1. Procédé pour détecter un bactériophage dans un échantillon comme indicateur d'une qualité hygiénique de l'échantillon, le procédé comprenant les étapes de :
a. combinaison de l'échantillon et d'un milieu dans un premier récipient pour former un premier mélange, le premier récipient ayant une sortie de premier récipient, le milieu comprenant : (i) des nutriments pour la croissance d'une bactérie fécale ; (ii) un agent d'induction d'enzyme intra-cellulaire capable d'induire la production, dans la bactérie fécale, d'une enzyme intracellulaire ; et (iii) un indicateur pour la présence de l'enzyme intracellulaire, l'enzyme intra-cellulaire étant libérée de la bactérie par la lyse de la bactérie par un bactériophage ;
b. entrée du premier mélange dans un second récipient pour former un second mélange, le second récipient comprenant une culture de la bactérie fécale, une entrée de second récipient et une sortie de second récipient ;
c. sortie du second mélange du second récipient, où l'entrée dans le second récipient et la sortie du second récipient surviennent sensiblement à la même vitesse, et où la bactérie fécale est retirée à une vitesse approximativement équivalente à une vitesse à laquelle la bactérie se divise ;
d. incubation du second mélange dans le second récipient ;
e. suivi du nombre de la bactérie dans le second mélange ;
f. régulation d'un débit du premier mélange dans le second récipient, et de la vitesse de retrait du second mélange du second récipient, par référence au nombre de bactérie dans le second mélange ; et
g. mesure de la présence de l'enzyme intracellulaire par l'effet de l'enzyme sur l'indicateur,
où la présence de l'enzyme bactérienne intracellulaire dans le second mélange indique la présence du bactériophage dans l'échantillon et où la présence du bactériophage est un indicateur pour la médiocre qualité hygiénique de l'échantillon.

2. Procédé selon la revendication 1 où la bactérie fécale comprend *Escherichia coli* et le bactériophage comprend un coliphage.

3. Procédé selon la revendication 2 où l'agent d'induction d'enzyme intracellulaire comprend l'isopropyl-β-D-thiogalactoside.

4. Procédé selon la revendication 2 où la bactérie fécale comprend *E. coli* dans une phase de croissance logarithmique.

5. Procédé selon la revendication 2 où l'indicateur comprend un indicateur fluorogène pour la présence de β-galactosidase.

6. Procédé selon la revendication 2 où l'indicateur est choisi dans le groupe consistant essentiellement en : 4-méthylombelliféryl-β-D-galactopyranoside ; O-nitrophényl-β-D-thiogalactoside ; 5-bromo-4-chloror-3-indoyl-β-D-galactoside ; et B-D-galactopyranoside de rouge de chlorophénol.

7. Procédé selon la revendication 1 où l'indicateur comprend une combinaison d'un indicateur coloré et d'un indicateur fluorescent.

8. Procédé selon la revendication 2 comprenant en outre le maintien de la bactérie fécale dans une phase de croissance logarithmique.

9. Procédé selon la revendication 1 où la régulation du débit comprend la mesure de la croissance cellulaire dans le second mélange et la fourniture d'une rétroaction à un dispositif de régulation d'écoulement.

10. Procédé selon la revendication 9 où la régulation du débit comprend la mesure de la densité optique du second mélange et la fourniture d'une rétroaction à un dispositif de régulation d'écoulement.

11. Procédé selon la revendication 1 comprenant en outre la mesure d'un changement détectable dans la sortie du second récipient.

12. Procédé selon la revendication 1 comprenant en outre la mesure d'un changement détectable dans le second récipient.

13. Procédé selon la revendication 1 comprenant en outre l'écoulement de l'échantillon en continu par une première entrée de récipient et dans le premier récipient.

14. Procédé selon la revendication 13 comprenant en outre l'écoulement de l'échantillon en continu, par une première entrée de récipient dans le premier récipient et l'écoulement du milieu en continu par une seconde entrée de récipient dans le premier récipient.

15. Procédé selon la revendication 1 où la détection du bactériophage comme indicateur de la qualité hygiénique de l'échantillon survient en moins de 8 heures de contact de l'échantillon avec le milieu.
